# EUROPEAN PATENT APPLICATION

(11) **EP 4 813 462 A1**
(43) Date of publication of application: **30.09.2026**
(21) Application number: 25166411.6
(22) Date of filing: 26.03.2025
(51) Int. Cl.: A61P 35/04, C07D 487/04, A61K 31/505

(54) **SALTS OF RIBOCICLIB HAVING IMPROVED STABILITY**

(71) Applicant: KRKA, d.d., Novo mesto, 8000 Novo mesto (SI)
(72) Inventor: STANFEL, Ursa, 8000 Novo mesto (SI); ZUPANCIC, Katja, 8000 Novo mesto (SI); TESTEN, Ana, 8000 Novo mesto (SI)
(74) Representative: Kutzenberger Wolff & Partner

(57) **Abstract**

The invention relates to physiologically acceptable salts of Ribociclib. In particular, the invention relates to Ribociclib ascorbate and Ribociclib caffeate, which have improved storage stability and shelf life at ambient condition compared to Ribociclib succinate salt or Ribociclib free base.

## Description

The invention relates to physiologically acceptable salts of Ribociclib. In particular, the invention relates to Ribociclib ascorbate and Ribociclib caffeate, which have improved storage stability and shelf life at ambient condition compared to Ribociclib succinate salt or Ribociclib free base.

Ribociclib has the systematic name 7-cyclopentyl-N,N-dimethyl-2-[[5-(1-piperazinyl)-2-pyridinyl]amino]-7H-pyrrolo-[2,3-d]pyrimidine-6-carboxamide (CAS Number: 1211441-98-3):

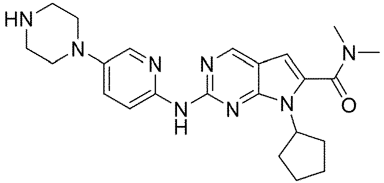

Ribociclib (LEE011; Novartis, ATC L01EP02) is a cyclin-dependent kinase 4/6 inhibitor (CDK4/6). Ribociclib drug product (Kisqali^{®}) was approved by FDA and EMA in 2017 and is commercially available as film coated, immediate release tablets containing 200 mg of Ribociclib. Each tablet contains 254.40 mg Ribociclib succinate (equivalent to 200 mg Ribociclib), microcrystalline cellulose, crospovidone type A, low-substituted hydroxypropyl cellulose, magnesium stearate and colloidal anhydrous silica as inactive ingredients, and is coated with PVA based film-coating composition.

Kisqali^{®} is approved in combination with an aromatase inhibitor for the adjuvant treatment of patients with hormone receptor (HR)-positive, human epidermal growth factor receptor 2 (HER2)-negative early breast cancer at high risk of recurrence. In pre- or perimenopausal women, or in men, the aromatase inhibitor should be combined with a luteinising hormone-releasing hormone (LHRH) agonist. FDA expanded the use of Kisqali^{®} in a co-package with Femara^{®} (letrozole) for the same indication (*https:*//*www.fda.gov*/*drugs*/*resources-information-approved-drugs*/*fda-approves-kisqali-aromatase-inhibitor-and-kisqali-femara-co-pack-early-high-risk-breast-cancer*)*.*

Kisqali^{®} is also approved for the treatment of women with HR-positive, HER2-negative locally advanced or metastatic breast cancer in combination with an aromatase inhibitor or fulvestrant as initial endocrine-based therapy, or in women who have received prior endocrine therapy. In pre- or perimenopausal women, the endocrine therapy should be combined with a LHRH agonist. Similarly, Kisqali^{®} Femara^{®} Co-Pack is indicated as initial endocrine-based therapy for the treatment of adults with HR-positive, HER2-negative advanced or metastatic breast cancer therapy.

Synthetic routes, salts and polymorphs of Ribociclib such as acetate, adipate, benzene sulfonate, citrate, fumarate, glutamate, glutarate, glycolate, hydrobromide, hydrochloride, malate, maleate, malo-nate, mesylate, oxalate, phosphate, p-toluene sulfonate, succinate, sulfate, tartrate, and trifluoroacetate are disclosed in e.g. WO 2010/020675 A1, WO 2012/064805 A1, WO 2016/091221 A1, WO 2016/166703 A1, WO 2018/051280 A1, WO 2019/040567 A1, WO 2019/062854 A1, WO 2019/082143 A1, WO 2019/130068 A1, WO 2019/150181 A1, WO 2019/167068 A1, WO 2020/152629 A1, WO 2020/222256 A1, WO 2022/207788 A2, WO 2024/115680 A1, WO 2025/041004 A1, CN 106 749 259 A, CN 108 245 486 A, CN 108 586 356 A, CN 109 400 612 A, and US 10,336,763 B1.

Ribociclib succinate, both as pharmacologically active ingredient and in finished dosage forms, undergoes transformation to N-nitroso Ribociclib, a substance flagged as potentially harmful due to the inclusion of the N-nitroso fragment in its structure:

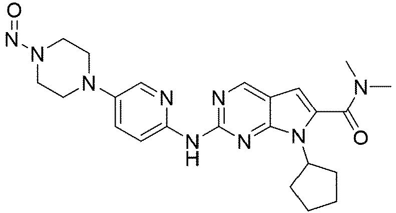

WO 2024/201299 A1 relates to a method of treating early breast cancer with Ribociclib in combination with aromatase inhibitor.

In the course of regulatory approval of treating early breast cancer, the Assessment Report of the Committee for Medicinal Products in Human Use (CHMP) (EMA/CHMP/512303/2024, 17 October 2024, Procedure No. EMEA/H/C/004213/II/0045) was adopted also with respect to N-nitroso Ribociclib impurity. In the adopted Assessment Report, it is stated that the N-nitroso Ribociclib impurity is present in trace amounts in Ribociclib succinate drug substance and in Ribociclib 200 mg film-coated tablet. N-nitroso Ribociclib impurity was determined to be mutagenic in an in vivo transgenic rodent gene mutation assay. N-nitroso Ribociclib impurity limits need to be controlled using the carcinogenic potency categorization approach by applying a maximum acceptable intake of 400 ng/day (category 3). The product information has been updated accordingly. This includes a restriction in shelf life and storage conditions from *"36 months without special conditions"* to *"refrigerated for up to 10 months at the pharmacy + up to 2 months below 25°C with the patient".*

Therefore, the physiologically acceptable salts of Ribociclib that are known from the prior art are not satisfactory in every respect and there is a demand for physiologically acceptable salts of Ribociclib that have advantages compared to these known salts.

Providing new solid forms of Ribociclib is an important aspect in pharmaceutical development, because the different solid forms affect the properties of the medicinal product such as thermodynamic stability, storage stability, solubility, bioavailability, tendency to form solvates, density, hygroscopicity, adhesiveness, electrical properties, mechanical properties (such as compressibility, friability, hardness, breaking strength, elasticity), optical properties (such as color, transparency, refraction), and the like. As with all pharmaceutical compounds and compositions, the chemical and physical properties of Ribociclib are important in its commercial development. These properties include, but are not limited to: (a) packing properties such as molar volume, bulk density and hygroscopicity, (b) thermodynamic properties such as melting temperature, vapor pressure and solubility, (c) kinetic properties such as dissolution rate and stability (including stability at ambient conditions, especially to moisture and under storage conditions), (d) surface properties such as surface area, wettability, interfacial tension and shape, (e) mechanical properties such as hardness, tensile strength, compactibility, handling, flow and blend; and (f) filtration properties. These properties can affect, for example, the processing and storage of Ribociclib and pharmaceutical compositions comprising Ribociclib. Salts, particularly crystalline salts, of Ribociclib that improve upon one or more of these properties relative to other salts and/or solid state forms of Ribociclib are desirable.

The properties of the final pharmaceutical dosage inter alia depend on the nature of different salts, their ansolvate/solvates and polymorphs thereof. Therefore, it is essential to investigate the properties and processes involved in the preparation and stabilization of the final pharmacologically active ingredient and its incorporation into the final pharmaceutical dosage form.

Different salts differ in physical properties (such as melting points, solubility). The variation appreciably influences the pharmaceutical properties, such as bioavailability, handling properties (hygroscopicity), dissolution rate and stability. In turn, such properties can significantly influence the processing characteristics and shelf life of the salt and consequently suitability for formulating the final dosage form. Additionally, selection of acid addition salt and polymorph is important to purify crude synthesized material with good yields to obtained final Ribociclib and its salts free of any toxic impurities.

It is an object of the invention to provide forms of Ribociclib and finished dosage forms containing Ribociclib that have advantages compared to the prior art. Ribociclib should have improved stability, should not be prone to formation of N-nitroso Ribociclib and should have satisfactory storage stability and shelf life at ambient conditions. Stability should satisfy the criteria set forth by EMA in Guideline on Stability Testing: Stability testing of existing active substances and related finished products (EMA/CVMP/QWP/709423/2022, 13 July 2023).

This object has been achieved with the subject-matter of the patent claims.

It has been surprisingly found that the initial levels of N-nitroso Ribociclib in both salts and finished dosage forms can be reduced, and the decomposition of Ribociclib salt to N-nitroso Ribociclib can be delayed. Thus, it is possible to prepare a pharmacologically active ingredient and a finished dosage form with an extended shelf life.

Based on the knowledge derived from the registrational changes implemented by the originator (Novartis), potential stability issues can be inferred that are associated with the formation of N-nitroso Ribociclib in the final pharmaceutical dosage form. It is thus imperative to control the levels of N-nitroso Ribociclib to ensure the required quality of the final pharmaceutical dosage form.

It has been surprisingly found that the selection of an appropriate acid addition salt is crucial in reducing the formation of undesirable N-nitroso impurities in the final pharmaceutical dosage form.

Stability testing indicated that Ribociclib succinate exhibited the poorest stability regarding N-nitroso formation. Initial contents of N-nitroso Ribociclib were significantly lower for Ribociclib ascorbate and for Ribociclib caffeate compared to Ribociclib succinate. In experimental tests, under the given conditions and comparing against Ribociclib succinate, N-nitroso Ribociclib formation was about 11 times lower with Ribociclib ascorbate, and about 15 times lower with Ribociclib caffeate. Additionally, accelerated stability testing revealed that Ribociclib ascorbate and Ribociclib caffeate maintain lower levels of N-nitroso Ribociclib compared to Ribociclib succinate, suggesting better stability profiles.

A first aspect of the invention relates to a physiologically acceptable salt of Ribociclib and
- ascorbic acid; or
- caffeic acid.

The physiologically acceptable salt according to the invention is a salt between a first salt component, namely Ribociclib, and a second salt component, namely either ascorbic acid or caffeic acid.

Preferably, the physiologically acceptable salt according to the invention is an acid addition salt wherein either ascorbic acid or caffeic acid serves as the acid and protonates Ribociclib. Protonated Ribociclib preferably forms the cation of the physiologically acceptable salt according to the invention, whereas the deprotonated ascorbic acid and caffeic acid, respectively, preferably forms the anion of the physiologically acceptable salt according to the invention.

In preferred embodiments, the physiologically acceptable salt according to the invention is Ribociclib ascorbate, i.e. a physiologically acceptable salt of Ribociclib (I) 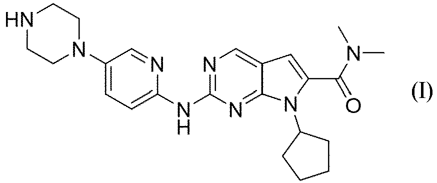 with ascorbic acid (II)

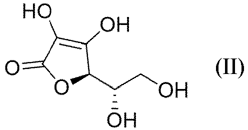

Preferably, the molar ratio of Ribociclib and ascorbic acid is 1:1.

In other preferred embodiment, the physiologically acceptable salt according to the invention is Ribociclib caffeate, i.e. a physiologically acceptable salt of Ribociclib (I) 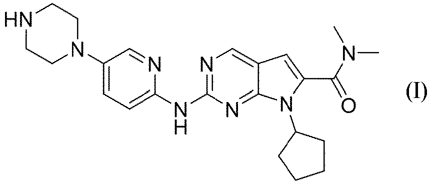 with caffeic acid (III)

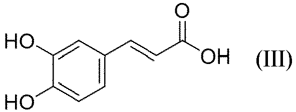

Preferably, the molar ratio of Ribociclib and caffeic acid is 1:1.

The salts according to the invention are so linked as to form a single general inventive concept because ascorbic acid and caffeic acid share certain common special technical features defining a contribution which the invention considered as a whole makes over the prior art.

From a functional perspective, ascorbic acid and caffeic acid are both acidic and thus capable of forming acid addition salts with Ribociclib. Further, ascorbic acid and caffeic acid both have a pronounced antioxidative effect and are typically both regarded as antioxidants.

From a structural perspective, ascorbic acid and caffeic acid both comprise two vicinal hydroxyl groups, which are each bonded to a carbon atom of a conjugated system. Physiologically acceptable acid addition salts with compounds comprising two vicinal hydroxyl groups, which are each bonded to a carbon atom of a conjugated system, are not known from the prior art.

As far as the undesired formation of N-nitroso Ribociclib (IV) is concerned 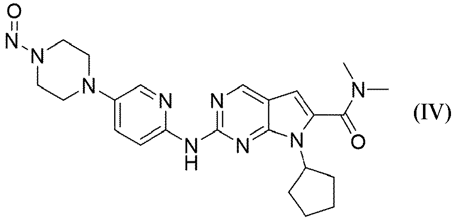 comparative experimental data demonstrate enhanced stability of the ascorbic acid salt of Ribociclib according to the invention and the caffeic acid salt of Ribociclib according to the invention compared to succinic salt of Ribociclib.

Preferably, the salt according to the invention is crystalline. However, amorphous salts are also contemplated.

In preferred embodiments, the salt according to the invention is a solvate, preferably a hydrate or a 2-propanol solvate.

In other preferred embodiments, the salt according to the invention is an ansolvate.

Another aspect of the invention relates to a pharmaceutical composition comprising a physiologically acceptable salt according to the invention as described above.

Preferably, the pharmaceutical composition according the invention comprises a filler/diluent.

Preferably, the pharmaceutical composition according the invention comprises a binder.

Preferably, the pharmaceutical composition according the invention comprises a disintegrant.

Another aspect of the invention relates to a pharmaceutical dosage form comprising a physiologically acceptable salt according to the invention as described above or a pharmaceutical composition according to the invention as described above.

Preferably, the pharmaceutical dosage form according to the invention is a tablet, more preferably a film-coated tablet.

Another aspect of the invention relates to a physiologically acceptable salt according to the invention as described above, a pharmaceutical composition according to the invention as described above, or a pharmaceutical dosage form according to the invention as described above, for use in the treatment of breast cancer, preferably early breast cancer, advanced breast cancer or metastatic breast cancer.

Another aspect of the invention relates to the use of a physiologically acceptable salt according to the invention as described above for the manufacture of a pharmaceutical composition according to the invention as described above, or of a pharmaceutical dosage form according to the invention as described above, for the treatment of breast cancer, preferably early breast cancer, advanced breast cancer or metastatic breast cancer.

Another aspect of the invention relates to a method for treating of breast cancer, preferably early breast cancer, advanced breast cancer or metastatic breast cancer, comprising administering an effective amount of a physiologically acceptable salt according to the invention as described above, a pharmaceutical composition according to the invention as described above, or a pharmaceutical dosage form according to the invention as described above, to a subject in need thereof.

Another aspect of the invention relates to a process for the preparation of a physiologically acceptable salt according to the invention as described above, a pharmaceutical composition according to the invention as described above, or a pharmaceutical dosage form according to the invention as described above.

Suitable processes for the preparation of Ribociclib caffeate and Ribociclib ascorbate are illustrated in the experimental section.

Ribociclib caffeate is preferably prepared from a solution of Ribociclib free base and caffeic acid in aqueous 2-propanol at elevated temperature. Crystallization is preferably induced by cooling and/or adding additional 2-propanol (see Example 2).

Ribociclib ascorbate is preferably prepared from a solution of Ribociclib free base and ascorbic acid in aqueous 2-propanol at elevated temperature. Crystallization is preferably induced by cooling and/or adding additional 2-propanol (see Example 3).

Examples 2 and 3 are for illustrative purpose how to prepare Ribociclib caffeate and Ribociclib ascorbate.

In general, suitable processes for the preparation of Ribociclib caffeate and Ribociclib ascorbate can alternatively or additionally involve any of the following steps:
(a) providing a solution or suspension of Ribociclib free base in a solvent or in a solvent mixture; preferably in a mixture of an organic solvent and water;
(b) providing a composition of the desired acid, i.e. either caffeic acid or ascorbic acid; preferably in solid form or dissolved in a solvent or in a solvent mixture; preferably dissolved in the same organic solvent or solvent mixture used in step (a), or dissolved in one of the solvents that is contained in the solvent mixture used in step (a), e.g. either in water or in an organic solvent;
(c) mixing the solution or suspension provided in step (a) and the composition provided in step (b) with one another;
(d) inducing precipitation, preferably crystallization, of the salt of Ribociclib with caffeic acid and ascorbic acid, respectively, from the mixture obtained in step (c).

In preferred embodiments, the molar ratio of the desired acid in the composition provided in step (b) to the Ribociclib free base in the solution or suspension provided in step (a) is greater than 1.00, preferably within the range of from 1.00 to 1.05.

Step (c) may involve adding the solution or suspension provided in step (a) to the composition provided in step (b). Alternatively, step (c) may involve adding the composition provided in step (b) to the solution or suspension provided in step (a).

In preferred embodiments, the mixture obtained in step (c) has a start temperature which can be an elevated temperature, i.e. a temperature above room temperature (i.e., above 23°C). This may be achieved by heating the mixture as such, and/or by heating the solution or suspension provided in step (a), and/or by heating the composition provided in step (b).

In preferred embodiments, the start temperature is within the range of from 20°C up to reflux temperature of the mixture obtained in step (c). In preferred embodiments, the start temperature is an elevated temperature, preferably within the range of from 50°C up to reflux temperature of the mixture obtained in step (c), more preferably within the range of from 50 to 60°C, still more preferably about 50°C.

In preferred embodiments, step (c) involves evaporation of solvent.

In preferred embodiments, step (d) involves cooling precipitation or crystallization, i.e. the temperature of the mixture obtained in step (c) is reduced from the start temperature, which may be an elevated temperature, to an end temperature, preferably at a controlled cooling rate, preferably to control oversaturation of the salt of Ribociclib with caffeic acid and ascorbic acid, respectively.

The end temperature may be an elevated temperature, room temperature (i.e. about 23°C), or below room temperature (i.e. below 23°C). In preferred embodiments, the end temperature is below 50°C, more preferably below 20°C, or below 10°C.

Preferably, the elevated temperature is within the range of from 50°C up to reflux temperature of the mixture obtained in step (c), and the end temperature is below 50°C, more preferably below 20°C, or below 10°C.

In preferred embodiments, step (d) involves precipitation or crystallization by adding an anti-solvent, i.e. the solubility of the salt of Ribociclib with caffeic acid and ascorbic acid, respectively, is reduced by adding an anti-solvent at a controlled addition rate, preferably to control oversaturation of the salt of Ribociclib with caffeic acid and ascorbic acid, respectively.

In preferred embodiments, step (d) involves both, cooling precipitation or crystallization as well as precipitation or crystallization by adding an anti-solvent.

In preferred embodiments, step (d) involves precipitation or crystallization by evaporation of solvent.

Organic solvents are not particularly limited any may be selected from halogenated hydrocarbons, alcohols, ketones, esters, and acetonitrile. Preferred organic solvents are 2-propanol, methanol, dichloromethane, and mixtures of thereof.

Antisolvents are likewise not particularly limited any may be selected from alkanes, alcohols, ethers, esters, and water. Preferred antisolvents are water, methanol, ethyl acetate, and mixtures thereof.

The nature of a given solvent to either function as solvent or antisolvent can be easily determined by routine tests.

Unless expressly stated otherwise, all dosages of Ribociclib are expressed as equivalent doses relative to the non-salt form of Ribociclib. Thus, the weight of ascorbic acid and caffeic acid, respectively, is not taken into account.

In early breast cancer, the preferred dose is 400 mg (e.g. two 200 mg film-coated tablets) of Ribociclib once daily for 21 consecutive days followed by 7 days off treatment, resulting in a complete cycle of 28 days. In patients with early breast cancer, the medicament should be taken until completion of 3 years of treatment or until disease recurrence or unacceptable toxicity occur.

In advanced or metastatic breast cancer, the preferred dose is 600 mg (e.g. three 200 mg film-coated tablets) of Ribociclib once daily for 21 consecutive days followed by 7 days off treatment, resulting in a complete cycle of 28 days. In patients with advanced or metastatic breast cancer, the treatment should be continued as long as the patient is deriving clinical benefit from therapy or until unacceptable toxicity occurs.

These comparatively high doses of Ribociclib emphasize the importance of keeping the level of N-nitroso Ribociclib (IV) as low as possible.

Further, levels of undesired N-nitroso Ribociclib (IV) can be further lowered by using substances with antioxidant properties in catalytic quantities during synthesis, preferably in the steps of purifying the intermediates.

The levels of N-nitroso Ribociclib (IV) may be further reduced by employing substances possessing potential antioxidant properties in catalytic amounts during the synthesis process, preferably during the purification steps of the intermediates.

Furthermore, it is preferred that the acid utilized is selected from the group consisting of ascorbic acid, caffeic acid, tartaric acid, lactic acid, citric acid, malic acid, and any combinations thereof.

### EXAMPLES

The following examples further illustrate the invention but are not to be construed as limiting its scope.

### Analytical methods:

Assay of N-Nitroso Ribociclib was determined with 1260 Infinity II + coupled with Sciex TQ 6500+.

Assay of N-Nitroso Ribociclib, LC-MS method, external standard method:

Column: xSelect CSH C18, (150 x 4.6) mm, 3.5 µ particles. Mobile phase A: 0,1 % solution of HCOOH (V/V). Mobile phase B= acetonitrile. Gradient:

| t (min) | Mobile phase A (%) | Mobile phase B (%) |
|---|---|---|
| 0 | 95 | 5 |
| 12 | 20 | 80 |
| 14 | 20 | 80 |
| 15 | 95 | 5 |
| 20 | 95 | 5 |

Divert valve during run:

| Divert time [min] | Valve State |
|---|---|
| 0.1 | To waste |
| 5.0 | To source |
| 7.5 | To waste |
| 20 | To source |

Flow: 1.0 mL/min. Injection volume: 5 µL. Column temperature: 30°C. Sample temperature: 7°C. Sample solvent: acetonitrile : water : formic acid = 700 : 300 : 1 (V/V). Run time - MS and LC: 20 minutes. Ionization mode: ESI, positive. Detection - MRM:

| Transition (m/z) | Collision energy (V) | Dwell (s) |
|---|---|---|
| 464 > 434 | 26 | 0.15 |
| 464 > 392 | 36 | 0.15 |

Ion source conditions:

| Ionization: | ESI+ |
|---|---|
| Dwell time | 150 msec |
| Curtain Gas (CUR) | 25 psi |
| Collision Gas (CAD) | 9 psi |
| IonSpray Voltage (IS) | 4000 V |
| Temperature (TEM) | 400°C |
| Ion Source Gas 1 (GS1) | 50 psi |
| Ion Source Gas 2 (GS2) | 50 psi |
| Declustering Potential (DP) | 70 V |
| Entrance Potential (EP) | 10 V |
| Collision Energy (CE) | See table of Detection - MRM |
| Collision Cell Exit Potential (CXP) | 10 V |

### HRMS spectra were obtained with Thermo_Vanquish coupled with HRMS LTQ Orbitrap.

LC-MS screening MS/MS method: Column: Supelcosil LC ABZ, 250 x 4,6 mm, 5 µm particles. Mobile phase A: solution of HCOOH, pH= 2.5. Mobile phase B: acetonitrile. Gradient:

| t (min) | Mobile phase A (%) | Mobile phase B (%) |
|---|---|---|
| 0 | 95 | 5 |
| 7 | 93 | 7 |
| 10 | 87 | 13 |
| 15 | 47 | 53 |
| 20 | 10 | 90 |
| 20.1 | 95 | 5 |
| 25 | 95 | 5 |

Flow: 1.4 mL/min. Injection volume: 20 µL. Column temperature: 60°C. UV detection: wavelength, 265 nm. Sample temperature: 7°C. Sample solvent: acetonitrile : water : formic acid = 700 : 300 : 1 (V/V). Run time - MS and LC: 25 minutes. Ionization mode: ESI, positive for Ribociclib cation. Ionization mode: ESI, negative for caffeic acid and ascorbic acid anion. FTMS Mass range: 100-1500 m/z. Ion source conditions:

| | |
|---|---|
| Source Voltage | 3500 V |
| Source Current | 62 µA |
| Vaporizer Temperature | 270°C |
| Sheath Gas Flow Rate | 60 arb |
| IAux Gas | 7 arb |
| Sweep Gas | 0 arb |
| Capillary Temperature | 300°C |

### Split ratio: no split

IR Identification: IR spectra were recorded with Perkin Elmer IR Spectrometer using KBr pellets KBr disc, r = 4 cm ⁻¹ (Ph. Eur. 2.2.24)

NMR Identification: NMR spectra were obtained with Avance NEO 600 MHz with Prodigy NMR sonde (Solvent DMSO-d6, temperature 25°C).

### Example 1 - preparation of Ribociclib free base (I):

Ribociclib free base was prepared according to the synthesis described in WO 2022/207788 A2.

### Example 2 - preparation of Ribociclib salt with caffeic acid (III):

Ribociclib base (3 g), 20.3 mL 50 % 2-propanol in water and 1.31 g of caffeic acid were charged into a 100 mL overhead glass reactor with pitch blade stirrer. The reaction mass was heated to 65°C and mixed until a clear solution was formed. The clear solution was cooled to 50°C and 20.3 mL 2-propanol was added dropwise. The obtained solution was cooled to room temperature and stirred further for 18 hours. The obtained suspension was filtered using vacuum filtration. Wet cake was washed with 6 mL of precooled 2-propanol and dried in a vacuum tray dryer at 50°C, p < 50 mbar. 3.94 g of Ribociclib caffeate was obtained.

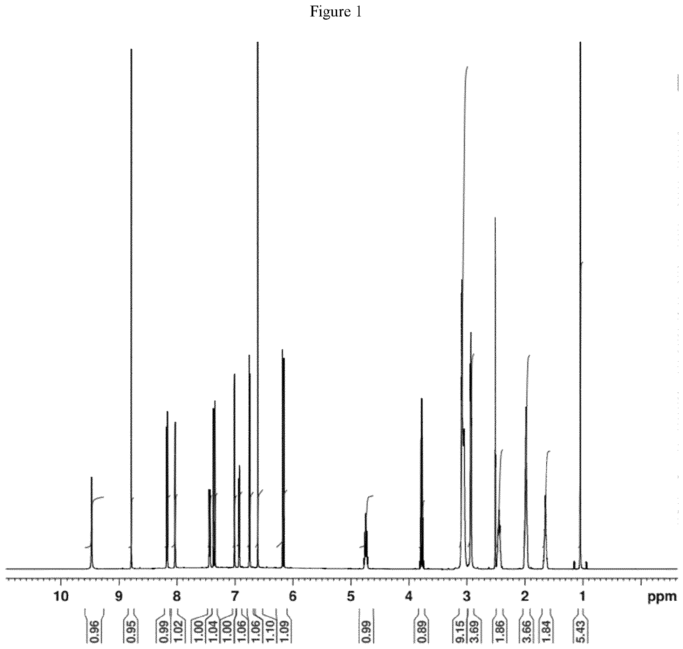

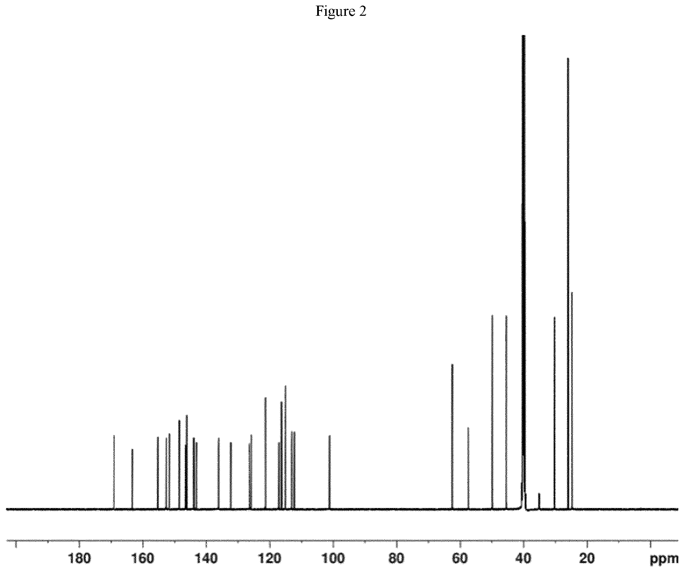

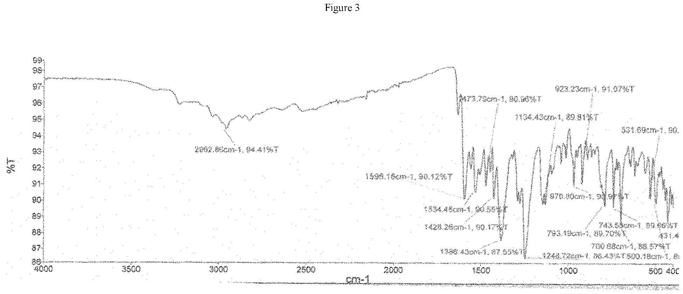

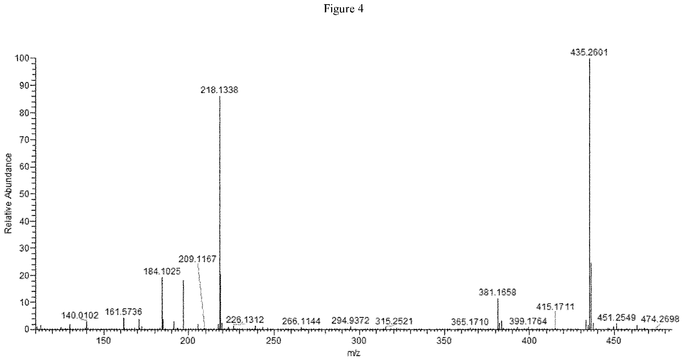

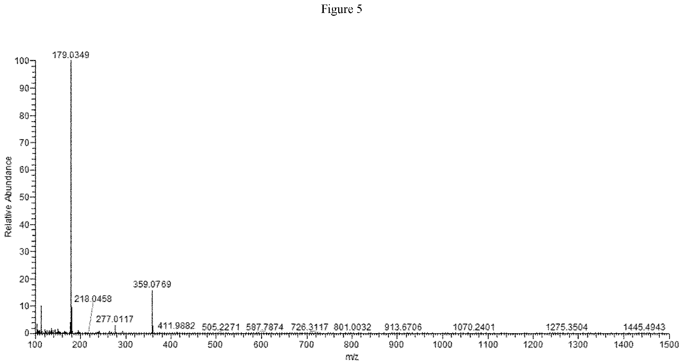

Figures 1 through 5 show 1H and 13C NMR spectra, IR spectrum and MS spectrum.

1H NMR (600 MHz, DMSO) δ 9.47 (s, 1H), 8.79 (s, 1H), 8.20 - 8.15 (m, 1H), 8.05 - 8.01 (m, 1H), 7.43 (dd, J = 9.1, 3.0 Hz, 1H), 7.36 (d, J = 15.8 Hz, 1H), 7.01 (d, J = 2.1 Hz, 1H), 6.93 (dd, J = 8.3, 2.1 Hz, 1H), 6.75 (d, J = 8.1 Hz, 1H), 6.60 (s, 1H), 6.17 (d, J = 15.8 Hz, 1H), 4.74 (p, J = 8.9 Hz, 1H), 3.11 - 3.07 (m, 10H), 2.93 (dd, J = 6.2, 3.7 Hz, 4H), 2.49 - 2.40 (m, 2H), 2.02 - 1.93 (m, 4H), 1.69 - 1.60 (m, 2H).

13C NMR (600 MHz, DMSO) δ 169.08, 163.36, 155.23, 152.58, 151.67, 148.50, 146.60, 146.13, 143.91, 143.15, 136.13, 132.29, 126.38, 125.81, 121.30, 117.06, 116.29, 115.03, 113.07, 112.24, 101.09, 78.39, 62.48, 49.77, 45.39, 40.39, 39.56, 30.19, 24.67, 2.98.

IR (cm⁻¹): 2963; 1596, 1534, 1474, 1428, 1386, 1249, 1134, 970, 923, 793, 743, 700, 532, 500.

MS: calculated for C₂₃H₃₁N₈O a [M+H]⁺: 435.2615 found: 435.2601.

MS: calculated for C₉H₇O₄ a [M-H]⁻: 179.0350 found: 179.0349.

### Example 3 - preparation of Ribociclib salt with ascorbic acid (II):

Ribociclib base (3 g), 20.3 mL 50 % 2-propanol in water and 1,28 g of ascorbic acid were charged into a 100 mL overhead glass reactor with pitch blade stirrer. The reaction mass was heated to 65°C and mixed until a clear solution was formed. The clear solution was cooled to 50°C and 20.3 mL 2-propanol was added dropwise. The obtained solution was cooled in steps to -5°C and at final temperature stirred for 3 hours. The obtained suspension was filtered using vacuum filtration. Wet cake was washed with 6 mL of precooled 2-propanol and dried in a vacuum tray dryer at 50°C, p < 50 mbar. 2.9 g of Ribociclib ascorbate was obtained.

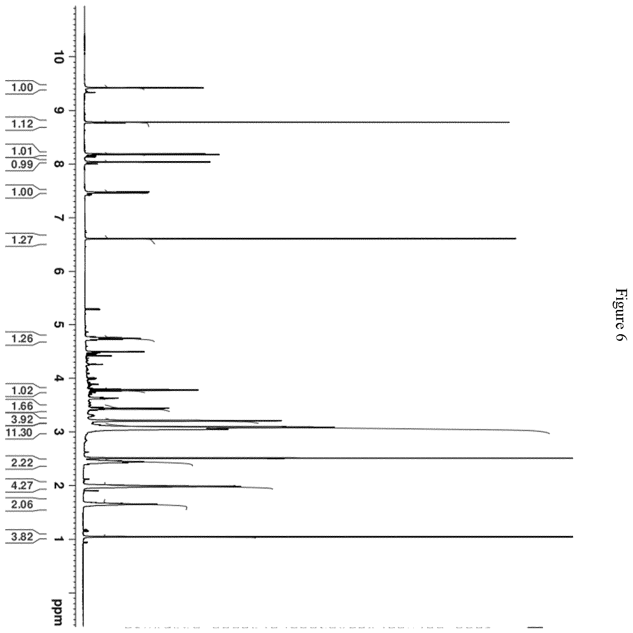

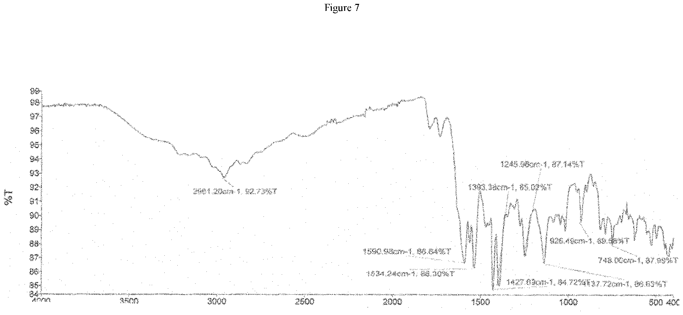

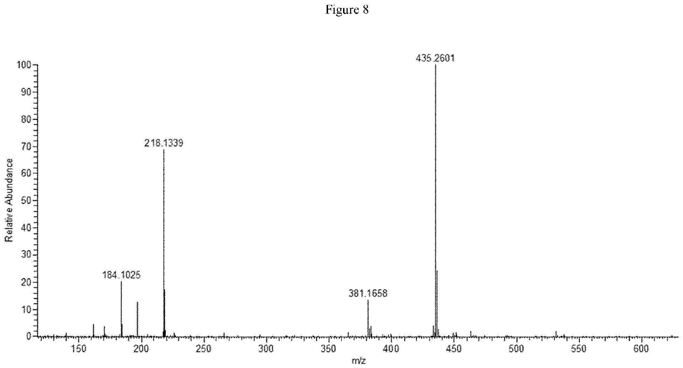

Figures 6 through 9 show 1HNMR spectra, IR spectrum and MS spectrum.

1H NMR (600 MHz, DMSO) δ 9.42 (s, 1H), 8.78 (s, 1H), 8.18 (d, J = 9.1 Hz, 1H), 8.04 (d, J = 3.0 Hz, 1H), 7.47 (dd, J = 9.1, 3.0 Hz, 1H), 6.61 (s, 1H), 4.74 (p, J = 9.0 Hz, 1H), 4.51 - 4.46 (m, 1H), 3.65 - 3.60 (m, 1H), 3.46 - 3.40 (m, 2H), 3.20 (dd, J = 7.0, 3.5 Hz, 4H), 3.11 - 3.03 (m, 10H), 2.49 - 2.41 (m, 2H), 1.98 (qd, J = 10.5, 5.6 Hz, 4H), 1.65 (qd, J = 7.1, 3.9 Hz, 2H).

IR (cm⁻¹): 2961; 1591, 1534, 1428, 1393, 1245, 1137, 926, 748.

MS: calculated for C₂₃H₃₁N₈O a [M+H]⁺: 435.2615 found: 435.2601.

MS: calculated for C₆H₇O₆ a [M-H]⁻: 175.0248 found: 175.0246.

### Example 4 - accelerated stability studies:

For the purpose of accelerated stability studies, 0.5 g of individual salt or free base of Ribociclib was precisely weighed and packed into two types of packaging: standard aluminum/triplex bags and aluminum/triplex bags flushed with nitrogen (N₂) atmosphere and supplemented with silica gel desiccant. The samples were subjected to controlled environmental conditions of 40°C and 75% relative humidity (RH).

The initial stability assessment involved testing the samples at two time points: one week and two weeks. The objective was to evaluate the impact of packaging and storage conditions on the chemical stability of the compounds. Quantitative mass spectrometry (QMS) was utilized to determine the concentration of N-nitroso Ribociclib (IV).

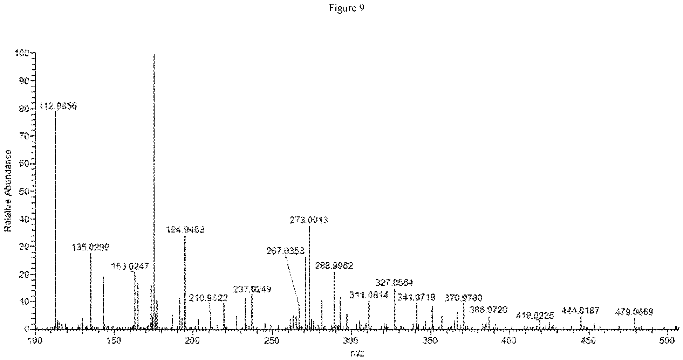

It has been surprisingly found that specific Ribociclib salts significantly reduce the initial assay of N-nitroso Ribociclib in the final pharmacologically active ingredient. The corresponding values [ppm] are presented in the table below and graphically represented in Figure 9:

| | Ribociclib free base | Ribociclib succinate | Ribociclib caffeate | Ribociclib ascorbate |
|---|---|---|---|---|
| Assay of N-nitroso Ribociclib [ppm] | 0.22 | 1.32 | 0.09 | 0.12 |

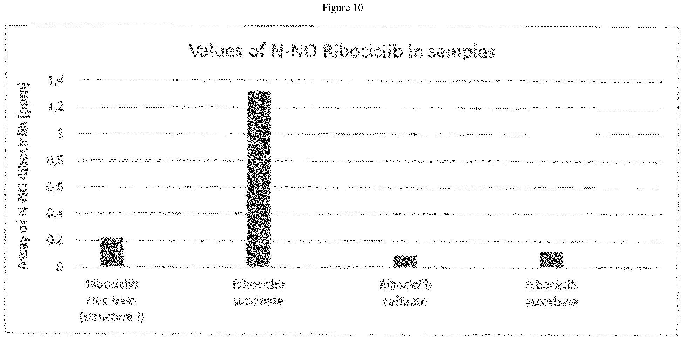

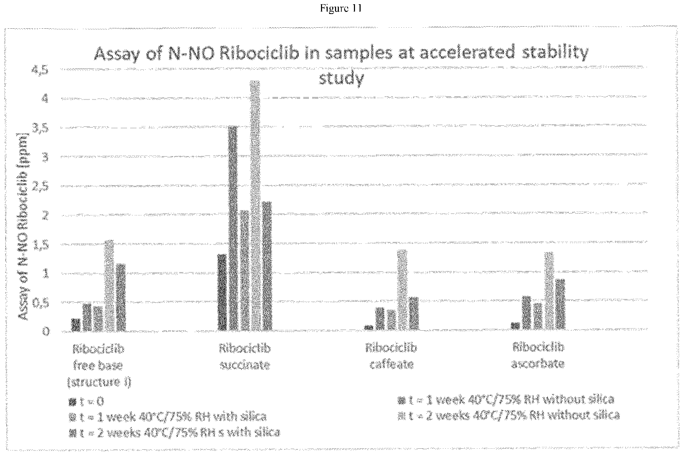

These salts were subjected to accelerated stability testing conditions, including elevated temperature and humidity. The results demonstrate that these salts possess inhibitory effects on the formation of N-nitroso Ribociclib, outperforming the reference succinate salt. This inhibition is crucial in mitigating potential stability issues and ensuring the quality and efficacy of the final dosage form. The corresponding values [ppm] are presented in the table below and graphically represented in Figure 10:

| 40°C/75% RH | t=0 | 1 week | | 2 weeks | |
|---|---|---|---|---|---|
| | | without silica | with silica | without silica | with silica |
| Ribociclib free base | 0.22 | 0.48 | 0.43 | 1.57 | 1.15 |
| Ribociclib succinate | 1.32 | 3.52 | 2.06 | 4.29 | 2.21 |
| Ribociclib caffeate | 0.09 | 0.39 | 0.34 | 1.38 | 0.56 |
| Ribociclib ascorbate | 0.12 | 0.58 | 0.45 | 1.33 | 0.86 |

These findings underscore the importance of selecting appropriate salt forms in the development process to prevent the formation of undesirable N-nitroso impurities. This approach not only enhances the stability of the pharmacologically active ingredient but also aligns with stringent regulatory requirements for drug safety and quality.

This discovery described is pivotal in enhancing the overall stability and safety profile of the drug.

### Example 5 - film coated tablets:

Film-coated pharmaceutical dosage forms (compressed tablets) in table of Example X below are prepared by dry granulation process. The manufacturing process includes five main steps: blending of intra-granular components, roller compaction, milling and blending with extra-granular excipients, compression, and film coating:

| Example X - [mg] | X-1 | X-2 | X-3 | X-4 | X-5 | X-6 | X-7 | X-8 | X-9 | X-10 | X-11 | X-12 |
|---|---|---|---|---|---|---|---|---|---|---|---|---|
| Ribociclib caffeate | 282.9 | / | 282.9 | / | 282.9 | / | 282.9 | / | 282.9 | / | 282.9 | / |
| Ribociclib ascorbate | / | 281.1 | / | 281.1 | / | 281.1 | / | 281.1 | / | 281.1 | / | 281.1 |
| Cellulose, Microcrystalline | 54.2 | 31.8 | 38.3 | 32.3 | 31.3 | 44 | 38.3 | 40.1 | 36.7 | 170.5 | 221 | 208 |
| Mannitol | / | 17.2 | / | / | / | / | 15 | 15 | / | / | / | / |
| Hydroxypropylcellulose, Low-Substituted | 48.1 | 48.1 | 46.2 | 44.6 | 32.2 | 48.1 | 44.6 | 44.6 | 44.6 | 111.7 | 90.7 | 96.9 |
| Crospovidone | 42 | / | / | 40 | 12.6 | / | / | / | / | 97.6 | 79.2 | 84.6 |
| Sodium Starch Glycolate | / | 44 | / | / | / | 54 | 43.6 | 43.6 | 43.6 | / | / | / |
| Croscarmellose Sodium | / | / | 40 | / | 41.2 | / | / | / | / | / | / | / |
| Magnesium Stearate | 14.8 | 14.8 | 15 | 14 | 12.8 | 14.8 | 15 | 15 | 14 | 34.5 | 28 | 29.7 |
| Silicon Dioxide, Anhydrous | 3 | 3 | 2.6 | 3 | 2 | 3 | 2.2 | 2.2 | 2.2 | 7.4 | 6 | 6.4 |
| HPMC film coating | 15 | 20 | 15 | 15 | 15 | 15 | 18.4 | / | / | 27.2 | 22.2 | 23.3 |
| Kollicoat^{®} IR | / | / | / | / | / | / | / | 18.4 | 16 | / | / | / |
| Total | 460 | 460 | 440 | 430 | 430 | 460 | 460 | 460 | 440 | 730 | 730 | 730 |

| Example X - [wt.-%] | X-1 | X-2 | X-3 | X-4 | X-5 | X-6 | X-7 | X-8 | X-9 | X-10 | X-11 | X-12 |
|---|---|---|---|---|---|---|---|---|---|---|---|---|
| Ribociclib caffeate | 61.5 | / | 64.3 | / | 65.8 | / | 61.5 | / | 64.3 | / | 38.8 | / |
| Ribociclib ascorbate | / | 61.1 | / | 65.4 | / | 61.1 | / | 61.1 | / | 38.5 | / | 38.5 |
| Cellulose, Microcrystalline | 11.8 | 6.9 | 8.7 | 7.5 | 7.3 | 9.6 | 8.3 | 8.7 | 8.3 | 23.4 | 30.3 | 28.5 |
| Mannitol | / | 3.7 | / | / | / | / | 3.3 | 3.3 | / | / | / | / |
| Hydroxypropylcellulose, Low-Substituted | 10.5 | 10.5 | 10.5 | 10.4 | 7.5 | 10.5 | 9.7 | 9.7 | 10.1 | 15.3 | 12.4 | 13.3 |
| Crospovidone | 9.1 | / | / | 9.3 | 2.9 | / | / | / | / | 13.4 | 10.8 | 11.6 |
| Sodium Starch Glycolate | / | 9.6 | / | / | / | 11.7 | 9.5 | 9.5 | 9.9 | / | / | / |
| Croscarmellose Sodium | / | / | 9.1 | / | 9.6 | / | / | / | / | / | / | / |
| Magnesium Stearate | 3.2 | 3.2 | 3.4 | 3.3 | 3.0 | 3.2 | 3.3 | 3.3 | 3.2 | 4.7 | 3.8 | 4.1 |
| Silicon Dioxide, Anhydrous | 0.7 | 0.7 | 0.6 | 0.7 | 0.5 | 0.7 | 0.5 | 0.5 | 0.5 | 1.0 | 0.8 | 0.9 |
| HPMC film coating | 3.3 | 4.3 | 3.4 | 3.5 | 3.5 | 3.3 | 4.0 | / | / | 3.7 | 3.0 | 3.2 |
| Kollicoat^{®} IR | / | / | / | / | / | / | / | 4.0 | 3.6 | / | / | / |
| Total | 100 | 100 | 100 | 100 | 100 | 100 | 100 | 100 | 100 | 100 | 100 | 100 |

Film-coated pharmaceutical dosage forms (compressed tablets) in table of Example Y below are prepared by wet granulation process. The manufacturing process includes five main steps: granulation solution preparation, wet granulation and drying of granulate, milling and blending with extra-granular excipients, compression, and film coating:

| Example Y - [mg] | Y-1 | Y-2 | Y-3 | Y-4 | Y-5 | Y-6 | Y-7 | Y-8 | Y-9 | Y-10 | Y-11 | Y-12 |
|---|---|---|---|---|---|---|---|---|---|---|---|---|
| Ribociclib caffeate | 282.9 | 282.9 | 282.9 | / | / | / | 282.9 | 282.9 | 282.9 | 282.9 | / | / |
| Ribociclib ascorbate | / | / | / | 281.1 | 281.1 | 281.1 | / | / | / | / | 281.1 | 281.1 |
| Povidone | / | / | / | 25.8 | 21.7 | / | 32.4 | / | 25.8 | 97.6 | / | 84.6 |
| Hydroxypropylcellulose | 27.1 | 21.6 | 25.8 | / | / | 17.3 | / | 21.6 | / | / | 90.7 | / |
| Water | q.s. | q.s. | q.s. | q.s. | q.s. | q.s. | q.s. | q.s. | q.s. | q.s. | q.s. | q.s. |
| Cellulose, Microcrystalline | 33.2 | 21.3 | 39.8 | 31.6 | 47.4 | 28.3 | 42.9 | 21.3 | 49.8 | 173.7 | 222.8 | 208 |
| Mannitol | / | 17.2 | / | / | / | 21.5 | / | 17.2 | / | / | / | / |
| Hydroxypropylcellulose, Low-Substituted | 42 | 45.4 | 46.2 | 46.2 | 43 | 43 | 45.4 | 45.4 | 36.2 | 111.7 | / | 96.9 |
| Crospovidone | 42 | / | 43 | 43 | / | / | / | / | 43 | / | 79.2 | / |
| Sodium Starch Glycolate | / | / | / | / | / | / | 45 | / | / | / | / | / |
| Croscarmellose Sodium | / | 44 | / | / | 44.5 | 44.5 | / | 44 | / | / | / | / |
| Magnesium Stearate | 14.8 | 9.2 | 12.9 | 12.9 | 12.9 | 14.9 | 9.2 | 9.2 | 12.9 | 34.5 | 28 | 29.7 |
| Silicon Dioxide, Anhydrous | 3 | / | 2.2 | 2.2 | 2.2 | 2.2 | 3 | / | 2.2 | 7.4 | 6 | 6.4 |
| HPMC film coating | 15 | / | 17.2 | 17.2 | 17.2 | 17.2 | 19.2 | 18.4 | / | 27.2 | 22.2 | 23.3 |
| Kollicoat^{®} IR | / | 18.4 | / | / | / | / | / | / | 17.2 | / | / | / |
| Total | 460 | 460 | 470 | 460 | 470 | 470 | 480 | 460 | 470 | 735 | 730 | 730 |

| Example Y - [wt.-%] | Y-1 | Y-2 | Y-3 | Y-4 | Y-5 | Y-6 | Y-7 | Y-8 | Y-9 | Y-10 | Y-11 | Y-12 |
|---|---|---|---|---|---|---|---|---|---|---|---|---|
| Ribociclib caffeate | 61.5 | 61.5 | 60.2 | / | / | / | 58.9 | 61.5 | 60.2 | 38.5 | / | / |
| Ribociclib ascorbate | / | / | / | 61.1 | 59.8 | 59.8 | / | / | / | / | 38.5 | 38.5 |
| Povidone | / | / | / | 5.6 | 4.6 | / | 6.8 | / | 5.5 | 13.3 | / | 11.6 |
| Hydroxypropylcellulose | 5.9 | 4.7 | 5.5 | / | / | 3.7 | / | 4.7 | / | / | 12.4 | / |
| Water | q.s. | q.s. | q.s. | q.s. | q.s. | q.s. | q.s. | q.s. | q.s. | q.s. | q.s. | q.s. |
| Cellulose, Microcrystalline | 7.2 | 4.6 | 8.5 | 6.9 | 10.1 | 6.0 | 8.9 | 4.6 | 10.6 | 23.6 | 30.5 | 28.5 |
| Mannitol | / | 3.7 | / | / | / | 4.6 | / | 3.7 | / | / | / | / |
| Hydroxypropylcellulose, Low-Substituted | 9.1 | 9.9 | 9.8 | 10.0 | 9.1 | 9.1 | 9.5 | 9.9 | 7.7 | 15.2 | / | 13.3 |
| Crospovidone | 9.1 | / | 9.1 | 9.3 | / | / | / | / | 9.1 | / | 10.8 | / |
| Sodium Starch Glycolate | / | / | / | / | / | / | 9.4 | / | / | / | / | / |
| Croscarmellose Sodium | / | 9.6 | / | / | 9.5 | 9.5 | / | 9.6 | / | / | / | / |
| Magnesium Stearate | 3.2 | 2.0 | 2.7 | 2.8 | 2.7 | 3.2 | 1.9 | 2.0 | 2.7 | 4.7 | 3.8 | 4.1 |
| Silicon Dioxide, Anhydrous | 0.7 | / | 0.5 | 0.5 | 0.5 | 0.5 | 0.6 | / | 0.5 | 1.0 | 0.8 | 0.9 |
| HPMC film coating | 3.3 | / | 3.7 | 3.7 | 3.7 | 3.7 | 4.0 | 4.0 | / | 3.7 | 3.0 | 3.2 |
| Kollicoat^{®} IR | / | 4.0 | / | / | / | / | / | / | 3.7 | / | / | / |
| Total | 100 | 100 | 100 | 100 | 100 | 100 | 100 | 100 | 100 | 100 | 100 | 100 |

## Claims

1. A physiologically acceptable salt of Ribociclib and
- ascorbic acid; or
- caffeic acid.

2. The salt according to claim 1, which is Ribociclib ascorbate.

3. The salt according to claim 1 or 2, wherein the molar ratio of Ribociclib and ascorbic acid is 1:1.

4. The salt according to claim 1, which is Ribociclib caffeate.

5. The salt according to claim 1 or 4, wherein the molar ratio of Ribociclib and caffeic acid is 1:1.

6. The salt according to any of the preceding claims, which is crystalline.

7. The salt according to any of the preceding claims, which is an ansolvate.

8. The salt according to any of claims 1 to 6, which is a solvate.

9. The salt according to claim 8, wherein the solvate is a 2-propanol solvate.

10. A pharmaceutical composition comprising a physiologically acceptable salt according to any of the preceding claims.

11. A pharmaceutical dosage form comprising a pharmaceutical composition according to claim 10, preferably a tablet, more preferably a film-coated tablet.
